# EUROPÄISCHE PATENTANMELDUNG

(11) **EP 0 205 023 A2**
(43) Veröffentlichungstag der Anmeldung: **17.12.1986**
(21) Anmeldenummer: 86107123.1
(22) Anmeldetag: 26.05.1986
(51) Int. Cl.: C07D 403/04, C07D 417/04, C07D 413/04, A01N 43/56, A01N 43/82, A01N 43/653

(54) **1-Aryl-4-heterocyclyl-pyrazole**

(30) Priorität: 07.06.1985 DE 3520332
(71) Anmelder: BAYER AG, 51368 Leverkusen (DE)
(72) Erfinder: Stetter, Jörg, Dr., D-5600 Wuppertal 1 (DE); Schallner, Otto, Dr., D-4019 Monheim (DE); Gehring, Reinhold, Dr., D-5600 Wuppertal 11 (DE); Lindig, Markus, Dr., D-4010 Hilden (DE); Santel, Hans-Joachim, Dr., D-5090 Leverkusen 1 (DE); Schmidt, Robert R., Dr., D-5060 Bergisch Gladbach 2 (DE)

(57) **Zusammenfassung**

Die Erfindung betrifft 1-Aryl-4-heterocyclyl-pyrazole der Formel (I) 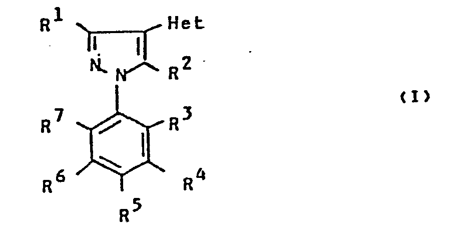 in welcher
R¹ für Wasserstoff oder Alkyl steht,
R² für Wasserstoff oder Halogen steht,
R³,R⁴,R⁵,R⁶ und R⁷ unabhängig voneinander jeweils für Wasserstoff, Cyano, Nitro, Halogen, Alkyl, Alkoxy, Alkoxycar. bonyl, Halogenalkyl, Halogenalkoxy oder für einen Rest -S(O)ₙ-R⁸ stehen,
mit der Maßgabe, daß mindestens einer der Reste R³, R⁴, R⁵, R⁶ oder R⁷ verschieden von Wasserstoff ist, wobei
R⁸ für Alkyl, Halogenalkyl, Amino, Alkylamino oder Dialkylamino steht und
n für eine Zahl 0, 1 oder 2 steht
   und

Het für einen gegebenenfalls durch Halogen, Nitro, Alkyl, Alkoxy, Alkylthio oder Halogenalkyl substituierten aromatischen Ein-Ring-Heterocyclus steht,
ein Verfahren zu ihrer Herstellung und ihre Verwendung als Herbizide.

## Beschreibung

Die Erfindung betrifft neue l-Aryl-4-heterocyclyl-pyrazole, ein Verfahren zu ihrer Herstellung und ihre Verwendung als Herbizide.

Es ist bereits bekannt, daß bestimmte 1-Aryl-pyrazole, wie beispielsweise das 4-Cyano-5-propionamido-1-(2,3,4-trichlorphenyl)-pyrazol herbizide Eigenschaften besitzen (vol. DE-OS 32 26 513).

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsgebieten völlig zufriedenstellend.

Es wurden neue 1-Aryl-4-heterocvclyl-pyrazole der allgemeinen Formel (I) 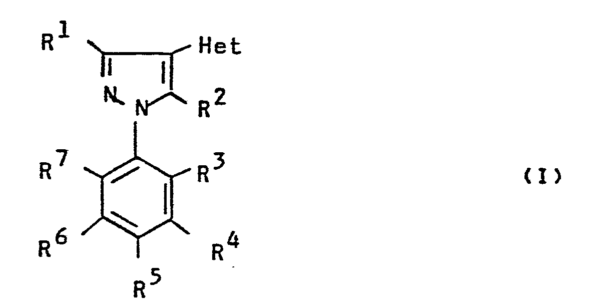 in welcher
R¹ für Wasserstoff oder Alkyl steht,
R² für Wasserstoff oder Halogen steht,
R³,R⁴,R⁵,R⁶ und R⁷ unabhängig voneinander jeweils für Wasserstoff, Cyano, Nitro, Halogen, Alkyl, Alkoxy, Alkoxycarbonyl, Halogenalkyl, Halogenalkoxy oder für einen Rest -S(O)ₙ-R⁸ stehen,
   mit der Maßgabe, daß mindestens einer der Reste R³, _{R}⁴, R⁵, R⁶ oder R⁷ verschieden von Wasserstoff ist, wobei
R⁸ für Alkyl, Halogenalkyl, Amino, Alkylamino oder Di- alk^{y}lamino steht und
n für eine Zahl 0, 1 oder 2 steht
   und
Het für einen gegebenenfalls durch Halogen, Nitro, Alkyl, Alkoxy, Alkylthio oder Halogenalkyl substituierten aromatischen Ein-Ring-Heterocyclus steht,

gefunden.

Weiterhin wurde gefunden, daß man die neuen 1-Aryl-4- heterocyclyl-pyrazole der allgemeinen Formel (I), 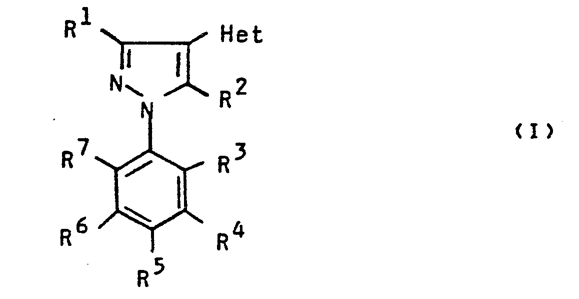 in welcher
R¹ für Wasserstoff oder Alkyl steht,
_{R}² für Wasserstoff oder Halogen steht,
_{R}³,_{R}⁴,_{R}⁵,R⁶ und R⁷ unabhängig voneinander jeweils für Wasserstoff, Cyano, Nitro, Halogen, Alkyl, Alkoxy, Alkoxycarbonyl, Halogenalkyl, Halogenalkoxy oder für einen Rest -S(O)ₙ-R⁸ stehen,

mit der Maßgabe, daß mindestens einer der Reste R³, R⁴, R⁵, R⁶ oder R⁷ verschieden von Wasserstoff ist, wobei
R⁸ für Alkyl, Haloaenalkyl, Amino, Alkylamino oder Di- alk^{y}lamino steht und
n für eine Zahl 0, 1 oder 2 steht
   und
   Het für einen gegebenenfalls durch Halogen, Nitro, Alkyl, Alkoxy, Alkylthio oder Halogenalkyl substituierten aromatischen Ein-Ring-Heterocyclus steht,

erhält, wenn man

5-Amino-l-aryl-4-heterocyclyl-pyrazole der Formel (II), in welcher 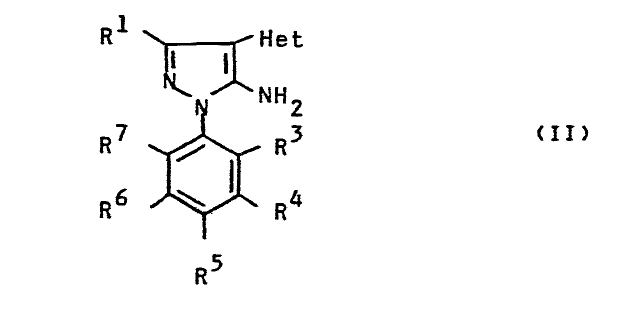 R¹,R³,R⁴,R⁵,R⁶,R⁷ und Het die oben angegebene Bedeutung haben,
mit einem anorganischen oder organischen Nitrit in Gegenwart eines Reaktionshilfsmittels und gegebenenfalls in Gegenwart einer Halogenwasserstoffsäure, sowie gegebenenfalls in Gegenwart eines Reduktionsmittels und gegebenenfalls in Gegenwart eines Verdünnungsmittels umsetzt.

Schließlich wurde gefunden, daß die neuen 1-Aryl-4- heterocyclyl-pyrazole der allgemeinen Formel (1) herbizide, insbesondere auch selektiv-herbizide Eigenschaften besitzen.

Uberraschenderweise zeigen die erfindungsgemäßen 1-Aryl-4-heterocyclyl-pyrazole der allgemeinen Formel (I) neben einer höheren herbiziden Wirksamkeit gegenüber Unkräutern auch eine erheblich verbesserte Selektivität gegenüber wichtigen Kulturpflanzen als die aus dem Stand der Technik bekannten 1-Aryl-pyrazole, wie beispielsweise das 4-Cyano-5-propionamido-1-(2,3,4-trichlorphenyl)-pyrazol, welche chemisch und wirkungsmäßig naheliegende Verbindungen sind.

Die erfindungsgemäßen 1-Aryl-4-heteracyclyl-pyrazole sind durch die Formel (I) allgemein definiert.

Bevorzugt sind Verbindungen der Formel (I), bei welchen
R^{l} für Wasserstoff oder für geradkettiges oder verzweigtes Alkyl mit 1 bis 6 Kohlenstoffatomen steht,
R² für Wasserstoff, Fluor, Chlor, Brom oder Iod steht,
R³,R⁴,R^{S},R⁶ und _{R}⁷ unabhängig voneinander jeweils für Wasserstoff, Cyano, Nitro, Halogen oder für jeweils geradkettiges oder verzweigtes Alkyl, Alkoxy oder Alkoxycarbonyl mit jeweils 1 bis zu 4 Kohlenstoffatomen im Alkylteil, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest -S(O)ₙ-R⁸ stehen, .

mit der Maßgabe, daß mindestens einer der Reste _{R}³, _{R}⁴, _{R}⁵, _{R}⁶ oder R⁷ verschieden von Wasserstoff ist, wobei
R⁸ für Amino, für jeweils geradkettiges oder verzweigtes Alkyl, Alkylamino oder Dialkylamino mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen steht und
n für eine Zahl 0, 1 oder 2 steht

und
Het für einen gegebenenfalls einfach oder mehrfach, gleich oder verschieden substituierten aromatischen fünf- oder sechsgliedrigen Ein-Ring- Heterocyclus steht, welcher ein bis drei gleiche oder verschiedene Heteroatome aus der Gruppe Stickstoff, Sauerstoff und Schwefel enthält, und über ein Kohlenstoff- oder ein Stickstoffatom verknüpft sein kann, wobei als Substituenten in Frage kommen:
   Halogen, Nitro, jeweils geradkettiges oder verzweigtes Alkyl, Alkoxy oder Alkylthio mit jeweils 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen.

Besonders bevorzugt sind die Verbindungen der Formel (I), bei welchen
R¹ für Wasserstoff, Methyl, Ethyl, n- oder i-Propyl, n-, i-, s- oder t-Butyl steht,
R² für Wasserstoff, Fluor, Chlor, Brom oder Iod steht,
R³,R⁴,R⁵,R⁶ und _{R}⁷ unabhängig voneinander jeweils für Wasserstoff, Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest -S(O)ₙ-R⁸ stehen,

mit der Maßgabe, daß mindestens einer der Reste R³, _{R}⁴, _{R}⁵, _{R}⁶ oder R⁷ verschieden von Wasserstoff ist, wobei
R⁸ für Methyl, Ethyl, Amino, Methylamino, Ethylamino, Dimethylamino, Diethylamino, Fluordichlormethyl, Difluorchlormethyl, Tetrafluorethyl, Trifluorchlorethyl oder Trifluormethyl steht,
n für eine Zahl 0, 1 oder 2 steht

und
Het für einen gegebenenfalls ein- bis dreifach, gleich oder verschieden durch Fluor, Chlor, Brom, Nitro, Methyl, Ethyl, n- und i-Propyl, Methoxy, Ethoxy, n-und i-Propoxy, Methylthio, Ethylthio, n- und i-Propylthio oder Trifluormethyl substituierten Heterocyclus der Formel 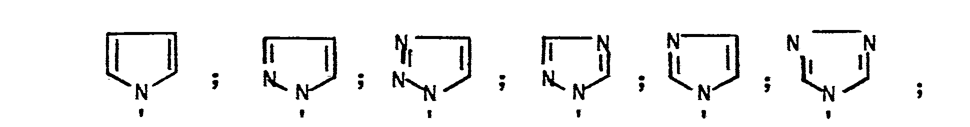 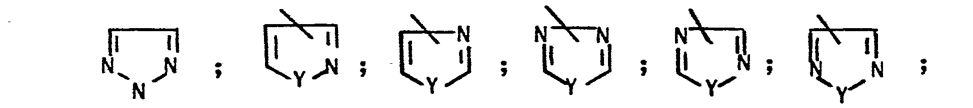 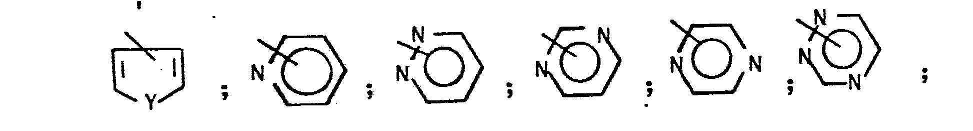 oder 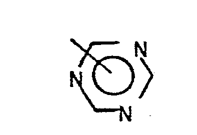
   steht, wobei
Y jeweils für Sauerstoff oder Schwefel oder einen N-Alkyl-Rest mit 1 bis 4 Kohlenstoffatomen steht.

Ganz besonders bevorzugt sind diejenigen Verbindungen der Formel (I), bei welchen
R¹ für Wasserstoff steht,
_{R}² für Wasserstoff, Chlor oder Brom steht,
R³,R⁴,R⁵,R⁶ und R⁷ unabhängig voneinander jeweils für Wasserstoff, Fluor, Chlor, Brom, Methyl, Ethyl, Isopropyl, Methoxy, Ethoxy, Trifluormethyl, Dichlorfluormethyl, Difluorchlormethyl, Trifluormethoxy oder für einen Rest -S(O)ₙ-R⁸ stehen, mit der Maßgabe, daß mindestens einer der Reste R³, _{R}⁴, R⁵, R⁶ oder R⁷ verschieden von Wasserstoff ist, wobei
R⁸ für Methyl, Trifluormethyl, Fluordichlormethyl oder Difluorchlormethyl steht und
n für eine Zahl 0, 1 oder 2 steht,
Het für einen Heterocyclus der Formel 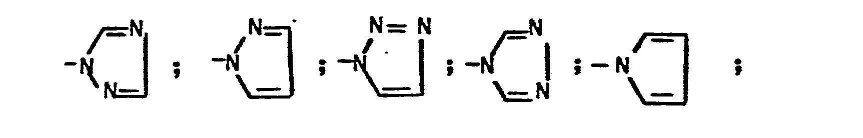 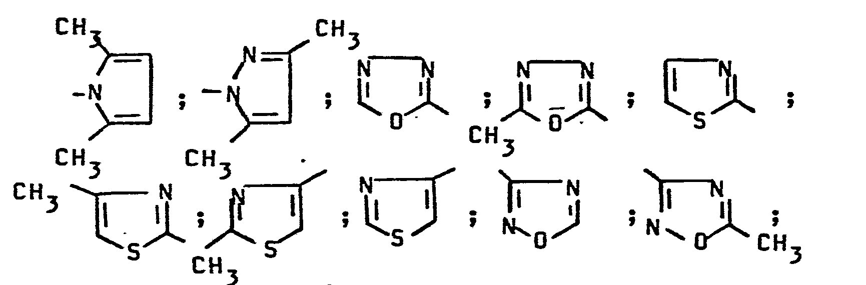 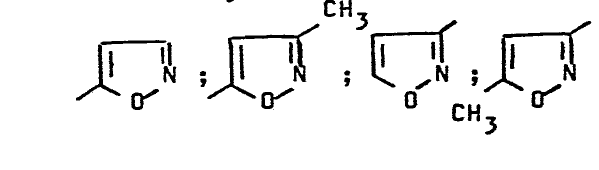 oder 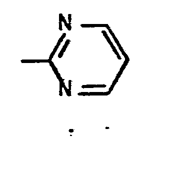 steht.

Im einzelnen seien außer den bei den Herstellungsbeispielen genannten Verbindungen die folgenden 1-Aryl-4- heterocyclyl-pyrazole der allgemeinen Formel (I) genannt:

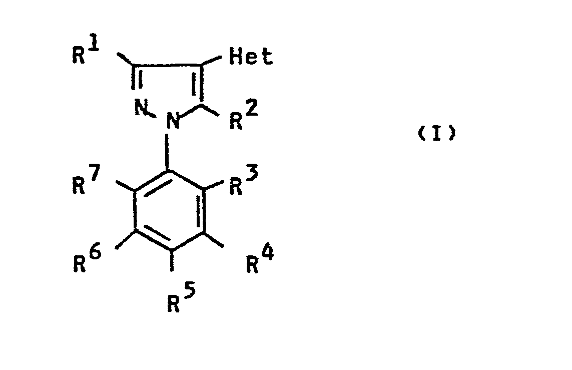

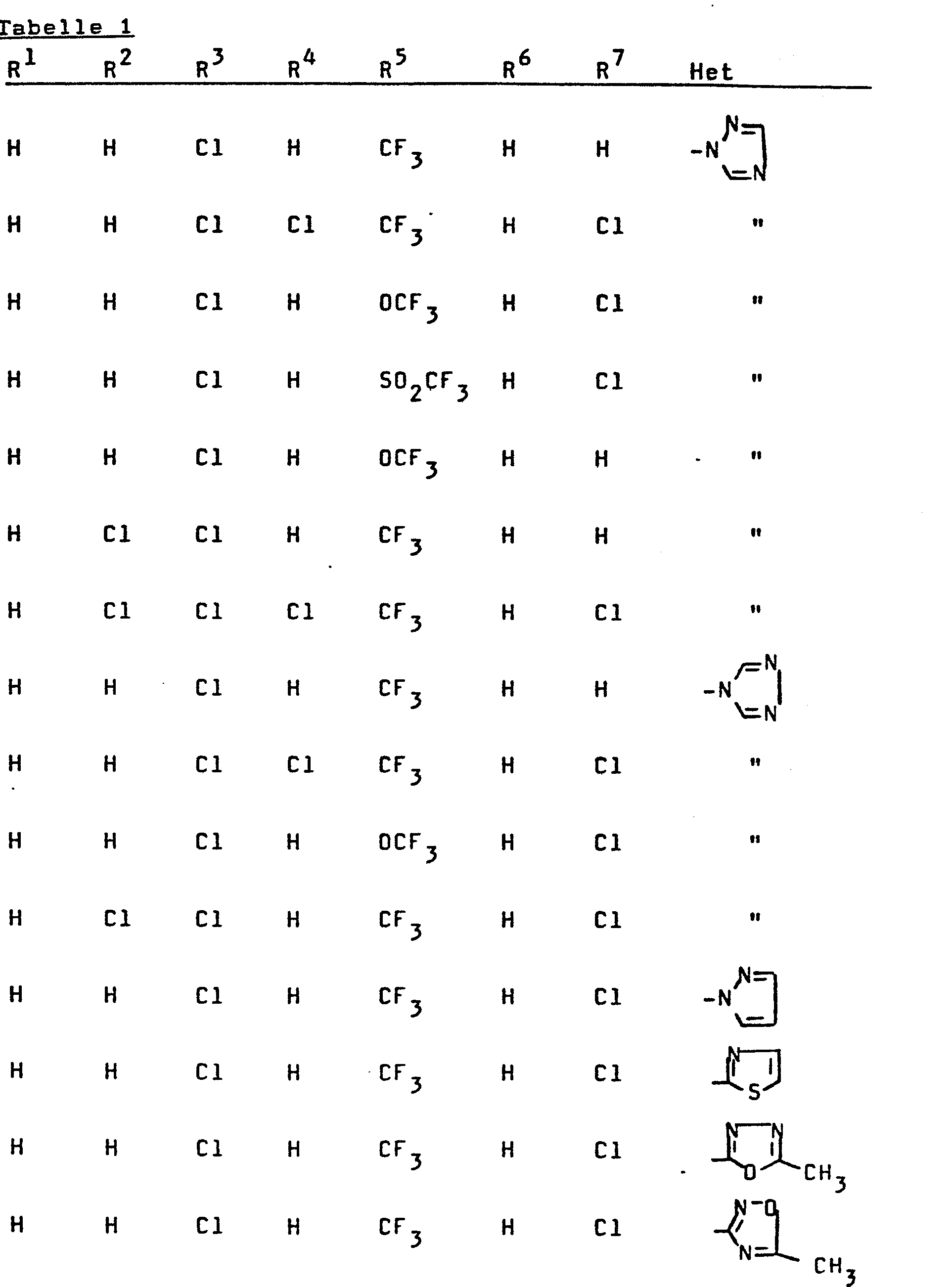

Verwendet man beispielsweise 5-Amino-4-(1,2,4-triazol-1-yl)-1-(2,6-dichlor-4-trifluormethylphenyl)-pyrazol als Ausgangsstoff und Natriumnitrit/Bromwasserstoffsäure als Reagenzien, so läßt sich der Reaktionsablauf des erfindungsgemäßen Verfahrens durch das folgende Formelschema darstellen:

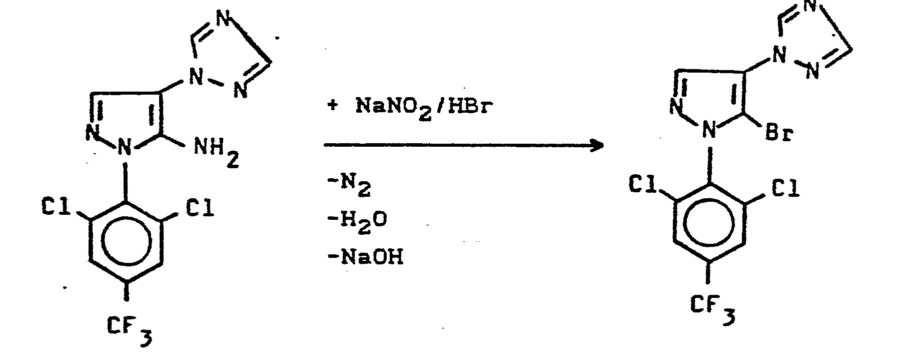

Die zur Durchführung des erfindungsgemäßen Verfahrens als Ausgangsstoffe benötigten 5-Amino-l-aryl-4-heterocyclyl- pyrazole sind durch die Formel (II) allgemein definiert. In dieser Formel (II) stehen R¹, R³, R⁴, R⁵, R⁶, _{R}⁷ und Het vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-4-heterocyclyl-pyrazole der Formel (II) sind noch nicht bekannt. Sie sind jedoch Gegenstand einer eigenen vorgängigen Patentanmeldung (DE-P 34 23 101 vom 22.6.1984).

Man erhält sie beispielsweise, wenn man Phenylhydrazine der Formel (III), 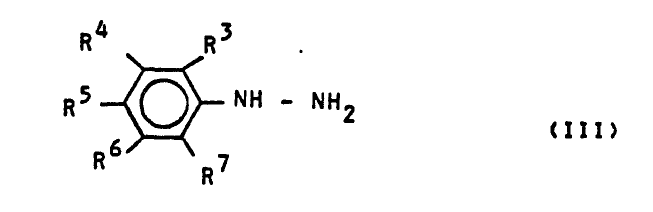 in welcher
R³,R⁴,R^{S},R⁶ und _{R}⁷ die oben angegebene Bedeutung haben,

mit Acrylnitril-Derivaten der Formel (IV), 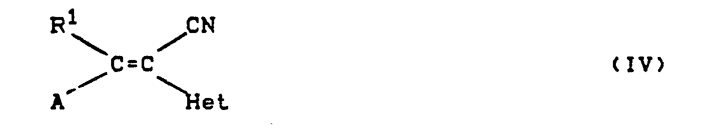 in welcher
R¹ und Het die oben angegebene Bedeutung haben und
A für Halogen, Hydroxy, Alkoxy oder Dialkylamino steht,

zunächst in einer 1. Stufe, gegebenenfalls in Gegenwart eines Verdünnungsmittels wie beispielsweise Ethanol oder Eisessig und gegebenenfalls in Gegenwart eines Reaktionshilfsmittels wie beispielsweise Natriumacetat bei Temperaturen zwischen -30°C und +50°C umsetzt zu den Phenyl-hydrazin-Derivaten der Formel (V), 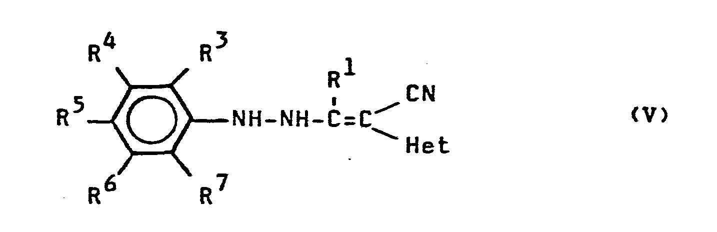 in welcher
R¹,R³,R⁴,R⁵,R⁶,R⁷ und Het die oben angegebene Bedeutung haben,

und diese in einer 2. Stufe, gegebenenfalls in Gegenwart eines Verdünnungsmittels, wie beispielsweise Ethanol oder Ethylenglykolmonomethylether, und gegebenenfalls in Gegenwart eines Katalysators, wie beispielsweise Schwefelsäure, bei Temperaturen zwischen +50_{.}C und +150°C .cyclisiert.

Die Umsetzung der Phenylhydrazine der Formel (III) mit Acrylnitril-Derivaten der Formel (IV) kann auch direkt in einem Reaktionsschritt ohne Isolierung der Zwischenstufe der Formel (V) gegebenenfalls in Gegenwart eines Verdünnungsmittels, wie beispielsweise Ethanol oder Ethylenglykolmonomethylether, bei Temperaturen zwischen +20°C und +150°C erfolgen.

Die Phenylhydrazine der Formel (III) sind größtenteils bekannt oder können nach bekannten Verfahren in einfacher, analoger Weise hergestellt werden (vgl. z.B. Houben-Weyl, "Methoden der organischen Chemie", Band X/2, S. 203, Thieme Verlag Stuttgart 1967), indem man beispielsweise die bekannten Aniline der Formel (VI), 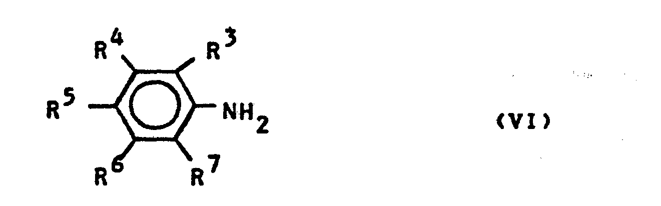 in welcher
R³,R⁴,R⁵,R⁶ und _{R}⁷ die oben angegebene Bedeutung haben,

mit Natriumnitrit, in Gegenwart einer Säure, wie beispielsweise Schwefelsäure, und dann mit Zinn-(II)-chlorid, ebenfalls in Gegenwart einer Säure, wie beispielsweise Salzsäure, bei Temperaturen zwischen -20°C und +80°C umsetzt.

Die Acrylnitril-Derivate der Formel (IV) sind Gegenstand einer eigenen vorgängigen Patentanmeldung (DE-P 34 23 101 vom 22.6.1984). Man erhält sie, wenn man Acetonitril-Derivate der Formel (VII), 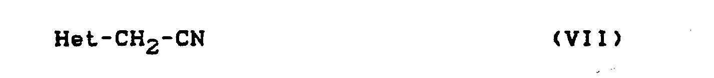 in welcher
Het die oben angegebene Bedeutung hat,

entweder mit Orthoestern der Formel (VIII), 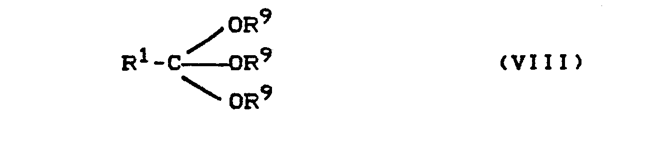 in welcher
R¹ die oben angegebene Bedeutung hat und
_{R}⁹ für Alkyl, insbesondere für Methyl oder Ethyl steht,

oder mit Amidacetalen der Formel (IX), 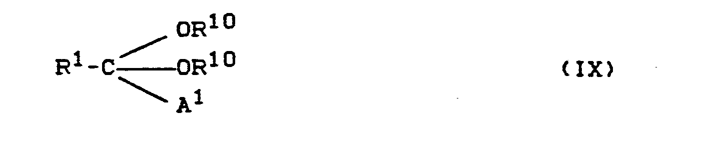 in welcher
R¹ die oben angegebene Bedeutung hat,
R¹⁰ für Alkyl, insbesondere für Methyl oder Ethyl steht und
A¹ für Dialkylamino, insbesondere für Dimethylamino steht,

gegebenenfalls in Gegenwart eines Verdünnungsmittels, wie beispielsweise Toluol, bei Temperaturen zwischen +80°C und +200°C umsetzt,
oder mit Estern der Formel (X), 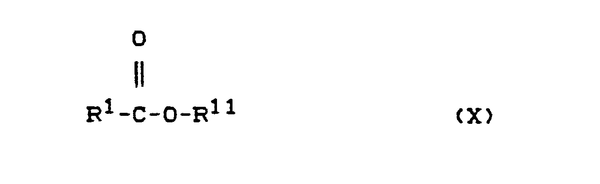 in welcher
R¹ die oben angegebene Bedeutung hat und
R¹¹ für Alkyl, insbesondere für Methyl oder Ethyl steht,

gegebenenfalls in Gegenwart eines Verdünnungsmittels, wie beispielsweise Methanol oder Ethanol, und gegebenenfalls in Gegenwart eines basischen Katalysators, wie beispielsweise Natriummethylat oder -ethylat, bei Temperaturen zwischen 0°C und 100°C umsetzt und die so erhältlichen Hydroxyverbindungen der Formel (IVa), 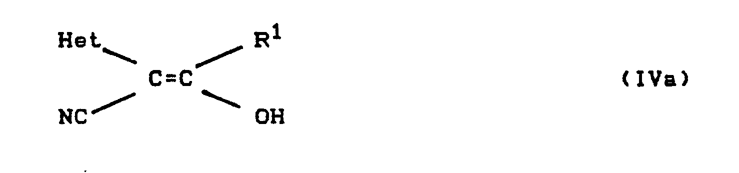 in welcher
R¹ und Het die oben angegebene Bedeutung haben,

in üblicher Weise mit Halogenierungsmitteln, wie Phosphorpentachlorid, Thionylchlorid oder Phosphortribromid, bei Temperaturen zwischen 0°C und +100°C substituiert.

Die Aniline der Formel (VI) und die Acetonitril-Derivate der Formel (VII) sind bekannt [vgl. z.B. Zh. org. Khim. 18, 463 (1982), C.A. 96, 181213x; DE-OS 31 29 429; Ber. dtsch. chem. Ges. 27, 3151 (1894)] oder lassen sich nach bekannten Verfahren in einfacher analoger Weise herstellen.

Die Orthoester der Formel (VIII), die Amidacetale der Formel (IX) und die Ester der Formel (X) sind allgemein bekannte Verbindungen der organischen Chemie.

Das erfindungsgemäße Verfahren wird in Gegenwart eines anorganischen oder organischen Nitrits durchgeführt. Als solche kommen alle für derartige Diazotierungsreaktionen üblichen Nitritverbindun^{g}en in Frage. Besonders bevorzugt verwendet man Alkalimetallnitrite, wie beispielsweise Natriumnitrit.

Wird das erfindungsgemäße Verfahren in Gegenwart einer Halogenwasserstoffsäure durchgeführt, so erhält man als Reaktionsprodukte l-Aryl-4-heterocyclyl-pyrazole der Formel (I), bei welchen R² für Halogen steht. Vorzugsweise verwendet man jeweils wäßrige Lösungen der Fluorwasserstoffsäure, Chlorwassersloffsäure, Bromwasserstoffsäure oder Iodwasserstoffsäure.

Das erfindungsgemäße Verfahren wird üblicherweise in Gegenwart eines Reaktionshilfsmittels durchgeführt. Als solch kommen insbesondere starke Mineralsäuren wie Schwefelsäure oder Phosphorsäure oder die oben auf^{g}e-führten Halogenwasserstoffsäuren in Frage, die in diesem Fall gleichzeitig als Reagenz und als Katalysator wirken.

Als Verdünnungsmittel zur Durchführung des erfindunasgemäßen Verfahrens kommen alle für derartige Diazotierunasreaktionen üblichen organischen und anorganischen Lösungsmittel in Frage. Vorzugsweise verwendet man die als Reak- tions^{p}artner eingesetzten Halogenwasserstoffsäuren im Überschuß oder andere wäßrige Säuren, wie beispielsweise Schwefelsäure, welche auch als Reaktionshilfsmittel dienen.

Das erfindungsgemäße Verfahren kann gegebenenfalls in Gegenwart eines geeigneten Reduktionsmittels durchgeführt werden. In diesem Fall erhält man als Reaktionsprodukte l-Aryl-4-heterocyclyl-pyrazole der Formel (I), bei welchen der Rest R² für Wasserstoff steht. Als Reduktionsmittel verwendet man in diesen Fällen besonders bevorzugt unterphosphorige Säure (H₃PO₂).

Die Reaktionstemperaturen können bei der Durchführung des erfindungsgemäßen Verfahrens in einem größeren Bereich variiert werden. Im allgemeinen arbeitet man bei Temperaturen zwischen -30°C und +60°C, vorzugsweise bei Temperaturen zwischen -20°C und +40°C.

Zur Durchführung des erfindungsgemäßen Verfahrens setzt man pro Mol an 5-Amino-1-aryl-4-heterocyclyl-pyrazol der Formel (II) im allgemeinen 1,0 bis 3,0 Mol, vorzugsweise 1,0 bis 1,8 Mol an Nitrit, gegebenenfalls 1,0 bis 20,0 Mol, vorzugsweise 1,0 bis 10,0 Mol an Halo^{g}enwasser- stoffsäure, gegebenenfalls 1,0 bis 50,0 Mol, vorzugsweise 1,0 bis 20,0 Mol an Reduktionsmittel und gegebenenfalls 1,0 bis 20,0 Mol, vorzugsweise 1,0 bis 10,0 Mol an als Reaktionshilfsmittel verwendete Mineralsäure ein.

Dabei setzt man üblicherweise der Reaktionsmischung bestehend aus 5-Amino-1-aryl-4-heterocyclyl-pyrazol. Mineralsäure, Verdünnungsmittel und Halogenwasserstoffsäure bzw. Reduktionsmittel, das Nitrit, welches gegebenenfalls in einem geeigneten Verdünnungsmittel gelöst ist, in kleinen Portionen zu.

Die Aufarbeitung und Isolierung der Reaktionsprodukte der Formel (I) erfolgt nach üblichen Methoden, z.B. durch Abfiltrieren von kristallinen Produkten oder durch Extraktion mit einem geeigneten organischen Lösungsmittel. Die Identifizierung erfolgt durch Schmelzpunkt und Protonen-Kernresonanz-Spektrum.

In einigen Fällen kann es sich als vorteilhaft erweisen, die erfindungsgemäßen Stoffe der Formel (I) auf einem alternativen Herstellungsweg zu synthetisieren:
Dazu baut man den Heterocyclus in 4-Position des Pyrazolringes aus einer geeigneten Vorstufe, wie beispielsweise einem 4-Amino-l-aryl-pyrazol der Formel (XI), in welcher 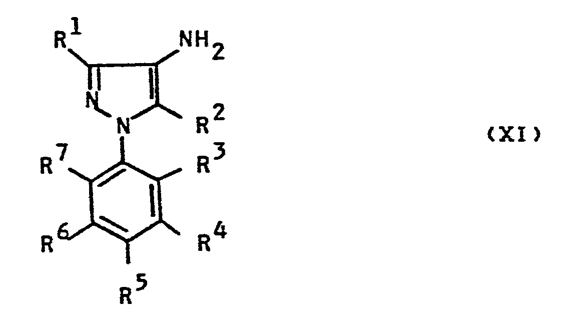
R¹,R²,R³,R⁴,R⁵,R⁶ und R⁷ die oben angegebene Bedeutung haben,

mit üblichen Reagenzien beispielsweise 1,4-Diketonen, wie Succindialdehyd oder N,N'-Bis-(farmyl)-hydrazin in Analogie zu bekannten Heterocyclensynthesen nachträglich auf (vgl. z.B. C. Ferri "Reaktionen der organischen Synthese", 1. Auflage, Thieme Verlag Stuttgart 1978, S. 698-812; vgl. auch Herstellungsbeispiele).

4-Amino-l-aryl-pyrazole der Formel (XI) erhält man beispielsweise durch Reduktion der entsprechenden 4-Nitro-1- aryl-pyrazole nach üblichen Verfahren z.B. mit Wasserstoff in Gegenwart eines Platinkatalysators und in Gegenwart eines geeigneten Verdünnungsmittels wie beispielsweise Methanol bei Temperaturen zwischen 0°C und 50°C. Die hierzu benötigten analogen 4-Nitro-l-aryl-pyrazole sind Gegenstand der eigenen vorgängigen Patentanmeldungen DE-P 35 01 323 vom 17.1.1985 und DE-P 35 09 567 vom 16.3.1985 und können beispielsweise aus geeigneten 1-Arylpyrazolen durch Nitrierung mit Salpetersäure gegebenenfalls in Gegenwart eines Verdünnungsmittels wie beispielsweise Eisessig und gegebenenfalls in Gegenwart eines Katalysators, wie beispielsweise Schwefelsäure oder Acetanhydrid, hergestellt werden (vgl. auch die Herstellungsbeispiele).

Die erfindungsgemäßen Wirkstoffe können als Defoliantsₜ Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikolvle Unkräuter der Gattunaen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium. Carduus. Sonchus, Solanum, Rorippa, Rotala, Lindernia, Lamium, Veronica, Abutilon, Emex, Datura, Viola, Galeopsis, Papaver, Centaurea.

Dikotvle Kulturen der Gattunaen: Gossypium, Glycine. Beta, Daucus, Phaseolus, Pisum, Solanum, Linum, Ipomoea, Vicia, Nicotiana. Lycopersicon, Arachis, Brassica, Lactuca, Cucumis, Cucurbita.

Monokotyle Unkräuter der Gattunaen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Aarostis, Alopecurus, Apera.

Monokotvle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono- und dikotyler Unkräuter in mono- und dikotylen Nutzpflanzenkulturen wie beispielsweise Zuckerrüben, Sojabohnen, Mais oder Weizen einsetzen.

In entsprechend verringerten Aufwandmengen zeigen die erfindungsgemäßen Wirkstoffe darüber hinaus eine gute fungizide Wirksamkeit und lassen sich zur Bekämpfung von Reiskrankheiten wie beispielsweise gegen den Erreger der Reisfleckenkrankheit (P^{y}ricularia or^{y}zae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise her^{g}e-stellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln: als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide: 4-Amino-3-methyl-6-phenyl-l,2,4-triazin-5(4H)-on zur Un- krautbekämpfun^{g} in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit 4-Amino-6-t-butyl-3-ethylthio-1,2,4-triazin-5(4H)-on; 2,4-Dichlorphenoxyessigsäure; 2,4-Dichlorphenoxypropionsäure; (2-Methyl-4-chlorphenoxy)-essig⁻ säure: (4-Chlor-2-methylphenoxy)-propionsäure; Chloressi^{g-} säure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-[4-(3,5-Dichiorpyrid-2-yloxy)-phenoxy7-propionsäure-(2-benzyloxyethylester): -(trimethvlsilvlmethylester oder -(2,2-diethoxyethylester); Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N-[(4-methoxy-6-methyl-1,3,5-triazin-2-yl)-amino]-carbonyl -benzosulfonamid: N-Methyl-2-(l,3-benzthia- zol-2-yloxy)-aceianilid: N,N-Di-n-propul-thiocarbamid- säure-S-ethyiester; exo-l-Methyl-4-(1-methylethyl)-2-(2-methylnhenylmethoxy)-7-oxabicyclo-[2.2.1]-heptan; 2-[4-[[3-Chlor-5-(trifluormethyl)-2-pyridinyl]-oxy]-phenoxyl-propansäure bzw. -propansäureethylester; 5-Amino-4-chlor-2-phenyl-2,3-dihydro-3-oxo-pyridazin; 2-Chlor-4-ethylamino-6-isopropylamino-1,3,5-triazin; 6-Chlor-3-phenyl-pyridazin-4-yl-S-octyl-thiocarbonat; 2-[5-Methyl-5-(1-methylethyl)-4-oxo-2-imidazolin-2-yl]-3-chinolincarbonsäure; Ethyl-2-[[(4-chlor-b-methoxy-2-pyrimidinyl)-aminacarbonyl]-aminosulfonyl]-benzoat; [(4-Amino-3,5-dichlor-6-fluor-2-pyridinyl)-oxy]-essigsäure bzw. -1-methylheptylester sind von Vorteil.

Einige Mischungen zeigen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittel sind möglich.

Die Wirkstoffe können als solche, in Form ihrer Formulierungen oder den daraus durch weiteres Verdünnen bereiteten Anwendungsformen, wie gebrauchsfertige Lösungen. Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

### Beispiel 1

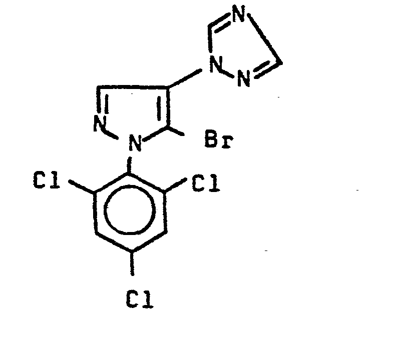

In eine Anschlämmung von 33 g (0,1 Mol) 5-Amino-4-(1,2,4-triazol-1-yl)-1-(2,4,6-trichlorphenyl)-pyrazol in 200 ml (4,5 Mol) wäßriger 50 %iger Bromwasserstoffsäure tropft man unter Rühren bei -5° bis 0°C eine Lösung aus 12 g (0,17 Mol) Natriumnitrit in 30 ml Wasser. Nach beendeter Zugabe rührt man unter langsamen Erwärmen auf +40°C weitere 3 Stunden und saugt dann den aus der erkalteten Reaktionsmischung ausgefallenen Feststoff ab, schlämmt ihn erneut in Wasser auf, neutralisiert mit Natriumhydrogencarbonat, saugt ab und trocknet den so erhaltenen Feststoff.

Man erhält 32 g (81 % der Theorie) an 5-Brom-4-(1,2,4-triazol-1-yl)-1-(2,4,6-trichlorphenyl)-pyrazol vom Schmelzpunkt 123°-125°C.

### Herstellung der Ausgangsverbindungen:

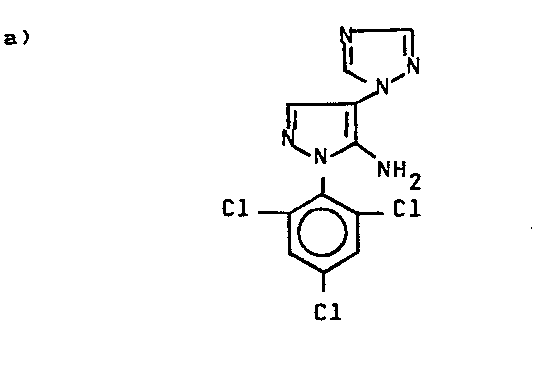

1,6 g (0,075 Mol) 2,4,6-Trichlor-phenyl-hydrazin und 16 g (0,075 Mol) 3-Dimethylamino-2-(1,2,4-triazol-1-yl)-acrylnitril-hydrochlorid in 200 ml Ethanol werden unter Rühren für 5 Stunden auf 75°C bis 78°C erhitzt. Zur Aufarbeitung wird das Lösungsmittel abdestilliert, der Rückstand mit Wasser angerieben und auf Ton getrocknet.

Man erhält 23,7 g (96 % der Theorie) an 5-Amino-4-(l,2,4-triazol-1-yl)-1-(2,4,6-trichlorphenyl)-pyrazol vom Schmelzpunkt 170-174°C.

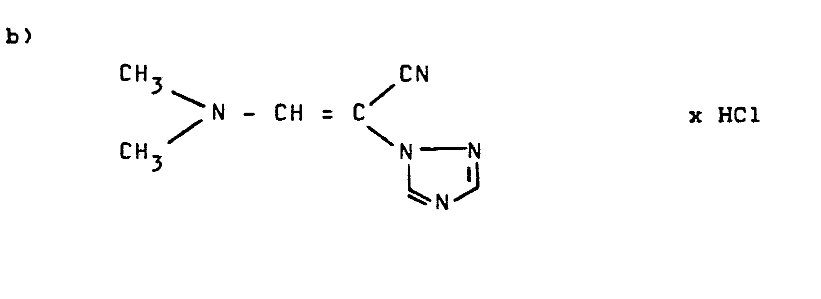

108 ^{g} (1 Mol) 1,2,4-Triazol-1-yl-acetonitril und 160 g (1,35 Mol) N,N-Dimethylformamid-dimethylacetal werden 4 Stunden unter Rückfluß erhitzt: das entstandene Methanol wird abdestilliert bis die Innentemperatur auf 120°C an^{g}e-stiegen ist: der erkaltete Rückstand wird in 400 ml Ethanol gelöst und mit 400 ml einer 20-%igen ethanolischen Chlorwasserstofflösung versetzt. Der kristalline Niederschlag wird abgesaugt, mit wenig Ethanol nachgewaschen und auf Ton getrocknet.

Man erhält 163 g (82 % der Theorie) an 3-Dimethylamino-2-(1,2,4-triazol-1-yl)-acrylnitril-hydrochlorid vom Schmelzpunkt 200-204°C.

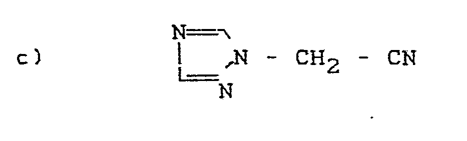

Zu einer Mischung aus 69 ^{g} (1 Mol) 1,2,4-Triazol, 150 g (1,08 Mol) gemahlenem Kaliumcarbonat und 500 ml Acetonitril gibt man tropfenweise unter Rühren bei 65-70°C während 30 Minuten 76 g (1 Mol) Chloracetonitril, rührt nach beendeter Zugabe weitere 3 Stunden bei 70°C, filtriert aus der erkalteten Reaktionsmischung den unlöslichen Niederschlag ab, engt das Filtrat im Vakuum ein und destilliert den Rückstand im Hochvakuum.

Man erhält 75 g (69 % der Theorie) an 1.2.4-Triazol-1-yl- acetonitril vom Siedepunkt 85°C bei 0,01 mbar, welches beim Erkalten erstarrt und einen Schmelzpunkt von 55°C bis 58°C besitzt.

### Beispiel 2

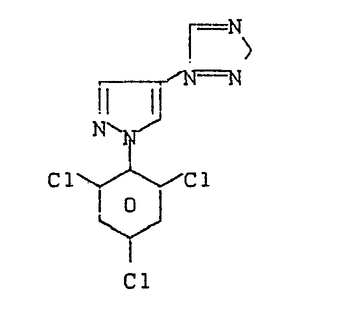

In eine Mischung aus 80 ml Wasser und 30 ml (0,55 Mol) 98-%ige Schwefelsäure tropft man bei 0°C 80 ml (0,9 Mol) 50-%ige unterphosphorige Säure und trägt dann ebenfalls bei 0°C unter Rühren 16,5 g (0,05 Mol) 5-Amino-4-(1,2,4-triazol-1-yl)-1-(2,4,6-trichlorphenyl)-pyrazol ein, rührt 30 Minuten nach und tropft dann eine Lösung aus 5 ^{g} (0,072 Mol) Natriumnitrit in 12 ml Wasser zu. Nach beendeter Zugabe rührt man 16 Stunden bei Raumtemperatur nach, gießt die Mischung in Wasser, extrahiert mit Toluvl. wäscht die Toluolphase nacheinander mit Wasser, verdünnter Natronlauge und wieder mit Wasser, trocknet über Magnesiumsulfat und entfernt das Lösungsmittel im Vakuum. Der ölige Rückstand kristallisiert beim Verreiben mit Ligroin und wird nach dem Absaugen auf Ton aetrocknet.

Man erhält 9,2 g (58,5 % der Theorie) an 4-(1,2,4-Triazol-1-yl)-1-(2,4,6-trichlorphenyl)-pyrazol vom Schmelzpunkt 112°-114°C.

### Beispiel 3

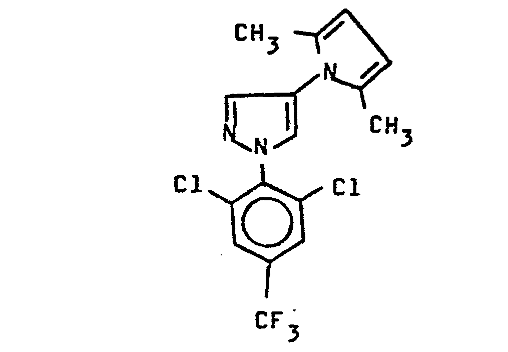

11,8 g (0,04 Mol) 4-Amino-1-(2,6-dichlor-4-trifluormethylphenyl)-pyrazol, 5,2 g (0,045 Mol) Hexan-2,5-dion und 0,3 g (0,001 Mol) p-Toluolsulfonsäure in 150 ml Toluol werden 20 Stunden über einen Wasserabscheider unter Rückfluß erhitzt, zur Aufarbeitung wird die Reaktionsmischung im Vakuum eingeengt, der Rückstand durch Anreiben mit Ligroin kristallisiert, abgesaugt und auf Ton getrocknet.

Man erhält 9,6 g (64 X der Theorie) an 1-(2,6-Dichlor-4-trifluormethylphenyl)-4-(2,5-dimethyl-pyrazol-1-yl)-pyrazol vom Schmelzpunkt 185°C.

### Herstellung der Ausgangsverbindungen

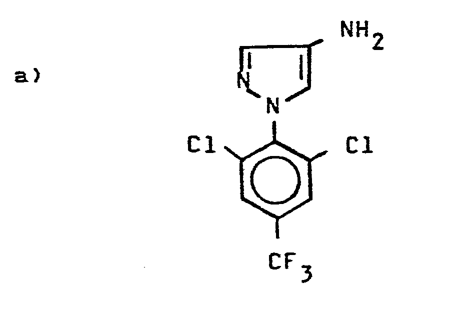

196 g (0,6 Mol) 1-(2,6-Dichlor-4-trifluormethylphenyl)-4-nitro-pyrazol und 8 g Platin-Kohle in 600 ml Methanol werden im Autoklaven bei Raumtemperatur 3 Stunden lang mit 12 bar Wasserstoffüberdruck hydriert. Zur Aufarbeitung filtriert man den Katalysator ab, engt das Filtrat im Vakuum ein, kristallisiert den Rückstand durch Anreiben mit Ligroin, saugt ab und trocknet auf Ton.

Man erhält 143 g (81 % der Theorie) an 4-Amino-1-(2,6-dichlor-4-trifluormethylphenyl)-pyrazol vom Schmelzpunkt 100°C bis 103°C.

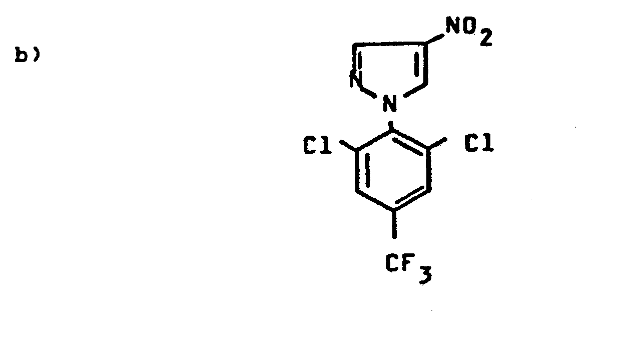

Zu 31,5 g (0,112 Mol) 1-(2,6-Dichlor-4-trifluormethyi- phenyl)-pyrazol in 150 ml 96-Xiger Schwefelsäure gibt man bei Raumtemperatur 30 ml (0,714 Mol) 98-Xige Salpetersäure so zu, daß die Temperatur der Reaktionsmischung 40°C nicht übersteigt. Nach beendeter Zugabe rührt man 2 Stunden bei 50°C, kühlt dann auf ca. 15'C ab und gießt vorsichtig in ca. 1000 g Eis. Der Niederschlag wird abgesaugt, mit ca. 500 ml Wasser neutral gewaschen und bei 50°C im Vakuum getrocknet.

Man erhält 34,7 g (95 % der Theorie) an 1-(2,6-Dichlor-4-trifluormethylphenyl)-4-nitropyrazol vom Schmelzpunkt 91-97°C.

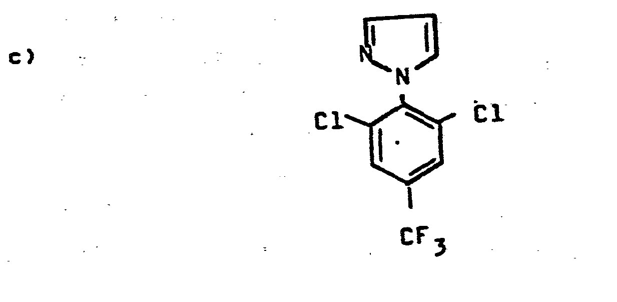

Zu 24,3 g (0,1 Mol) 2,6-Dichlor-4-trifluormethylphenyl- hydrazin in 100 ml Ethanol gibt man nacheinander 16,4 g (0,1 Mol) 1,1,3,3-Tetramethoxypropan und 5,1 g (0,05 Mol) 96-Xige Schwefelsäure und erhitzt für zwei Stunden auf Rückflußtemperatur. Nach dem Abkühlen der Reaktionsmischung neutralisiert man mit 5,3 g (0,05 Mol) Natriumcarbonat, rührt eine Stunde bei Raumtemperatur und engt dann im Vakuum ein. Der Rückstand wird in 250 ml Wasser aufgenommen und mit 200 ml Methylenchlorid extrahiert. Die organische Phase wird mit Wasser gewaschen, über Natriumsulfat getrocknet und im Vakuum vom Lösungsmittel befreit.

Man erhält 23,9 g (86,5 % der Theorie) an 1-(2,6-Dichlor-4-trifluormethylphenyl)-pyrazol vom Schmelzpunkt 83°C-85°C.

### Beispiel 4

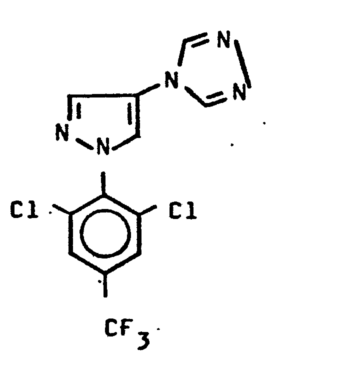

9 g (0,03 Mol) 4-Amino-1-(2,6-dichlor-4-trifluormethylphenyl)-pyrazol und 10 g (0,11 Mol) N,N'-Diformylhydrazin werden 8 Stunden lang auf 190°C erhitzt, das abgekühlte Gemisch in Wasser gegossen, abgesaugt, auf Ton getrocknet und aus Essigester umkristallisiert.

Man erhält 6,3 g (60 % der Theorie) an 1-(2,6-Dichlor-4-trifluormethylphenyl)-4-(1,3,4-triazol-1- yl)-pyrazol vom Schmelzpunkt 180°C.

In entsprechender Weise und gemäß den allgemeinen Angaben zur Herstellung erhält man die folgenden 1-Aryl-4-hetero- cyclyl-pyrazole der allgemeinen Formel (I):

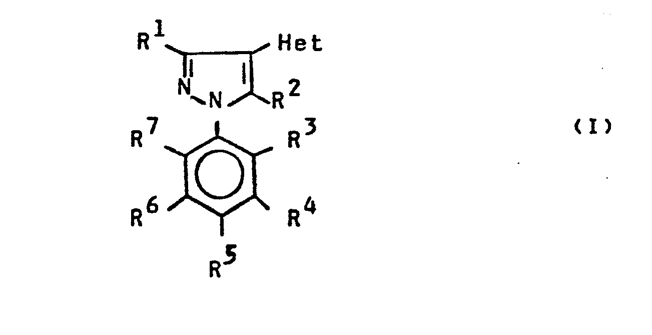

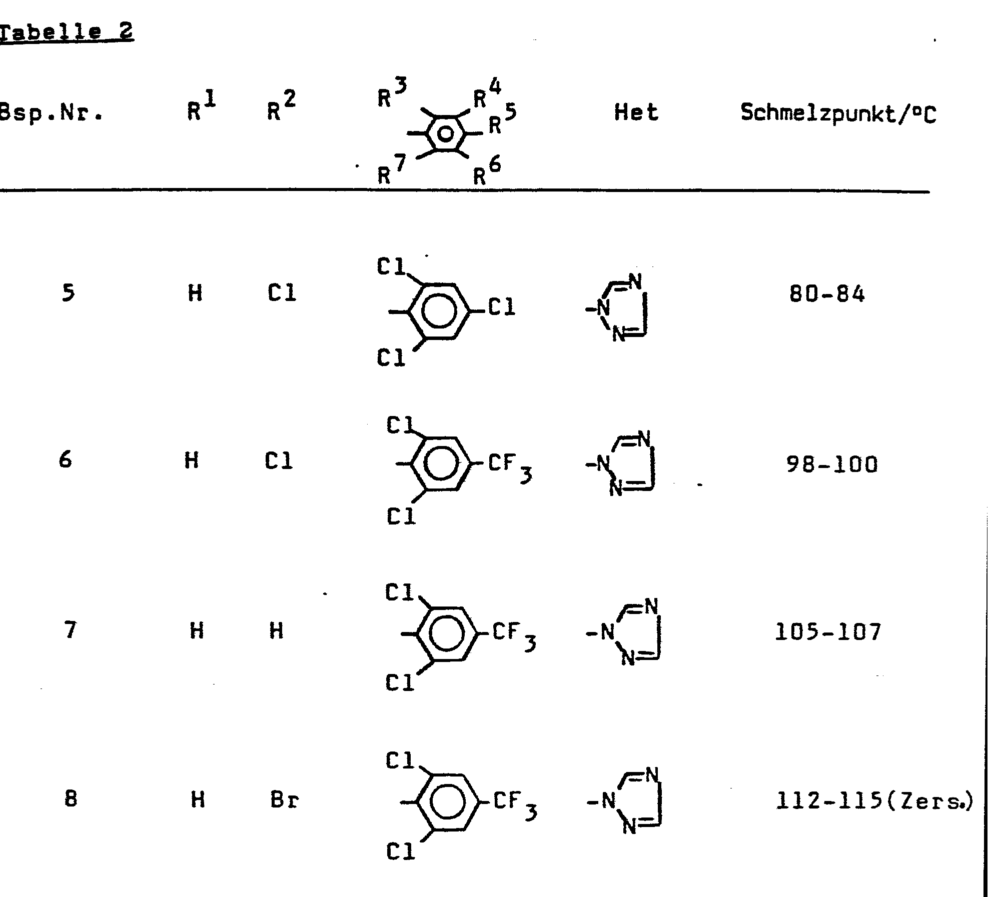

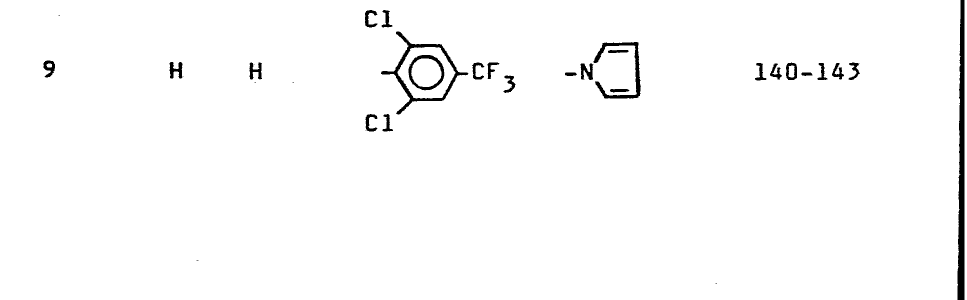

### Anwendunasbeispiele:

In den folgenden Anwendungsbeispielen wurde die nachstehend aufgeführte Verbindung als Vergleichssubstanz eingesetzt:

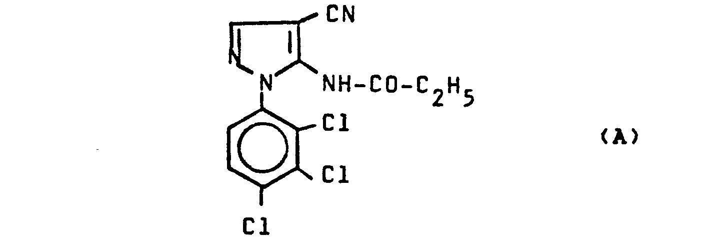

4-Cyano-5-propionamido-1-(2,3,4-trichlorphenyl)-pyrazol (bekannt aus DE-OS 32 26 513).

### Beispiel A

### Pre-emergence-Test / Gewächshaus

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der an^{g}e^{g}ebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Nutzpflanzenselektivität bei vergleichbarer Wirksamkeit gegenüber Unkräutern im Vergleich zum Stand der Technik zeigen in diesem Test z.B. die Verbindungen gemäß den Herstellungsbeispielen: 6 und 7.

### Beispiel B

### Post-emer^{g}ence-Test/Gewächshaus

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß den Herstellungsbeispielen: 6 und 7.

## Patentansprüche

1. 1-Aryl-4-heterocyclyl-pyrazole der Formel (I), 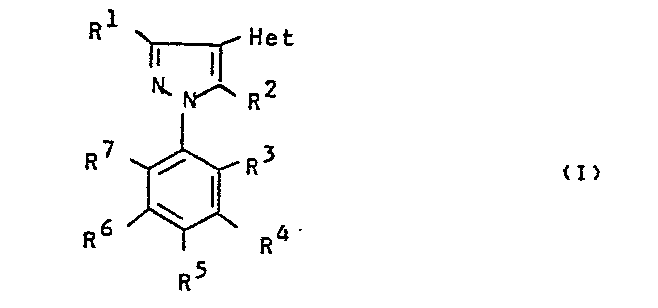 in welcher
R¹ für Wasserstoff oder Alkyl steht,
R² für Wasserstoff oder Halogen steht,
R³,R⁴,R⁵,R⁶ und R⁷ unabhängig voneinander jeweils für Wasserstoff, Cyano, Nitro, Halogen, Alkyl, Alkoxy, Alkoxycarbonyl, Halogenalkyl, Halo^{g}enalkoxy oder für einen Rest -S(O)ₙ-R⁸ stehen, mit der Maßgabe, daß mindestens einer der Reste R³, R⁴, R⁵, R⁶ oder R⁷ verschieden von Wasserstoff ist, wobei
R⁸ für Alkyl, Halogenalkyl, Amino, Alkylamino oder Dialk^{y}lamino steht und
n für eine Zahl 0, 1 oder 2 steht
und
Het für einen gegebenenfalls durch Halogen, Nitro, Alkyl, Alkoxy, Alkylthio oder Halogenalkyl substituierten aromatischen Ein-Ring-Heterocyclus steht.

2. 1-Aryl-4-heterocyclyl-pyrazole der Formel (I), in welcher 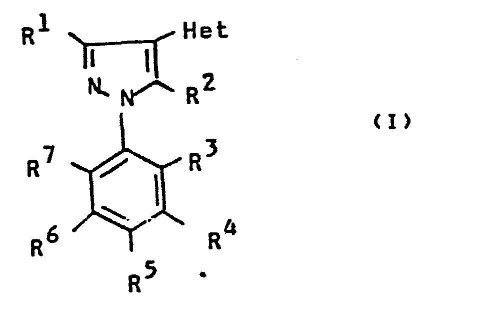
R¹ für Wasserstoff oder für geradkettiges oder verzweigtes Alkyl mit 1 bis 6 Kohlenstoffatomen steht,
R² für Wasserstoff, Fluor, Chlor, Brom oder Iod steht,
R³,R⁴,R⁵,R⁶ und _{R}⁷ unabhängig voneinander jeweils für Wasserstoff, Cyano, Nitro, Halogen oder für jeweils geradkettiges oder verzweigtes Alkyl, Alkoxy oder Alkoxycarbonyl mit jeweils 1 bis 4 Kohlenstoffatomen im Alkylteil, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest -S(O)ₙ-R⁸ stehen, mit der Maßgabe, daß mindestens einer der Reste _{R}³, _{R}⁴, _{R}⁵, R⁶ oder R¹ verschieden von Wasserstoff ist, wobei
R für Amino, für jeweils geradkettiges oder verzweigtes Alkyl, Alkylamino oder Dialkylamino mit jeweils 1 bis 4 Kohlenstoffatomen in den einzelnen Alkylteilen oder für geradkettiges oder verzweigtes Halogenalkyl mit 1 bis 4 Kohlenstoffatomen und 1 bis 9 gleichen oder verschiedenen Halogenatomen steht und
n für eine Zahl 0, 1 oder 2 steht
und
Het für einen gegebenenfalls einfach oder mehrfach, gleich oder verschieden substituierten aromatischen fünf- oder sechsgliedrigen Ein-Ring-Heterocyclus steht, welcher ein bis drei gleiche oder verschiedene Heteroatome aus der Gruppe Stickstoff, Sauerstoff und Schwefel enthält, und über ein Kohlenstoff- oder ein Stickstoffatom verknüpft sein kann, wobei als Substituenten in Frage kommen:
Halogen, Nitro, jeweils geradkettiges oder verzweigtes Alkyl, Alkoxy oder Alkylthio mit jeweils 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen.

3. l-Aryl-4-heterocyclyl-pyrazole der Formel (1) gemäß den Ansprüchen 1 und 2, in welcher
R¹ für Wasserstoff, Methyl, Ethyl, n- oder i-Propyl, n-, i-, s- oder t-Butyl steht,
R² für Wasserstoff, Fluor, Chlor, Brom oder Iod steht,
R³,R⁴,R⁵,R⁶ und R⁷ unabhängig voneinander jeweils für Wasserstoff, Cyano, Nitro, Fluor, Chlor, Brom, Iod, Methyl, Ethyl, n- und i-Propyl, n-, i-, s-und t-Butyl, für Methoxy, Ethoxy, Methoxycarbonyl, Ethoxycarbonyl, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest -S(O)ₙ-R⁸ stehen, mit der MaBgabe, daß mindestens einer der Reste R³, _{R}⁴, R⁵, R⁶ oder R⁷ verschieden von Wasserstoff ist, wobei
R⁸ für Methyl, Ethyl, Amino, Methylamino, Ethylamino, Dimethylamino, Diethylamino, Fluordichlormethyl, Difluorchlormethyl, Tetrafluorethyl, Trifluorchlorethyl oder Trifluormethyl steht,
n für eine Zahl 0, 1 oder 2 steht
und
Het für einen gegebenenfalls ein- bis dreifach, gleich oder verschieden durch Fluor, Chlor, Brom, Nitro, Methyl, Ethyl, n- und i-Propyl, Methoxy, Ethoxy, n- und i-Propoxy, Methylthio, Ethylthio, n- und i-Propylthio oder Trifluormethyl substituierten Heterocyclus der Formel 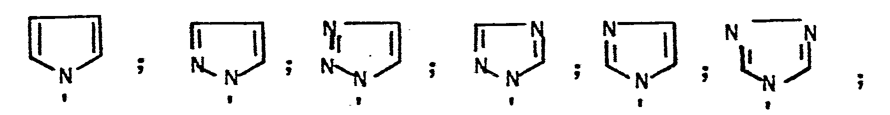 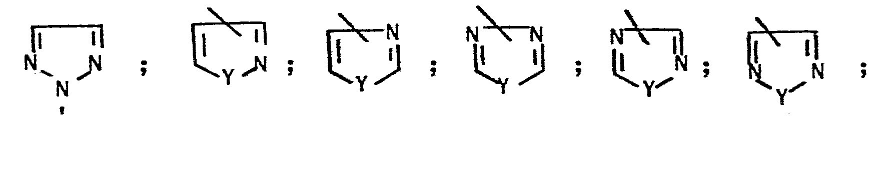 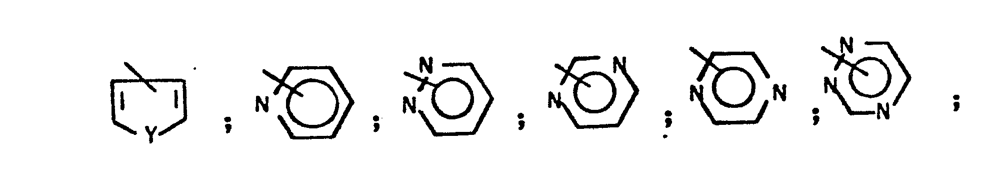 oder 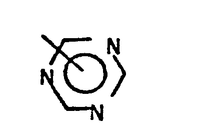 steht, wobei
Y jeweils für Sauerstoff oder Schwefel oder einen N-Alkyl-Rest mit 1 bis 4 Kohlenstoffatomen steht.

4. l-Aryl-4-heterocyclyl-pyrazole der Formel (I) gemäß den Ansprüchen 1 bis 2, in welcher
R¹ für Wasserstoff steht,
R² für Wasserstoff, Chlor oder Brom steht,
R³,R⁴,R⁵,R⁶ und R⁷ unabhängig voneinander jeweils für Wasserstoff, Fluor, Chlor, Brom, Methyl, Ethyl, Isopropyl, Methoxy, Ethoxy, Trifluormethyl, Dichlorfluormethyl, Difluorchlormethyl, Trifluormethoxy oder für einen Rest -S(O)ₙ-R⁸ stehen, mit der Maßgabe, daß mindestens einer der Reste _{R}³, _{R}⁴, _{R}⁵, _{R}⁶ oder R⁷ verschieden von Wasserstoff ist, wobei
R⁸ für Methyl, Trifluormethyl, Fluordichlormethyl oder Difluorchlormethyl steht und
n für eine Zahl 0, 1 oder 2 steht und
Het für einen Heterocyclus der Formel 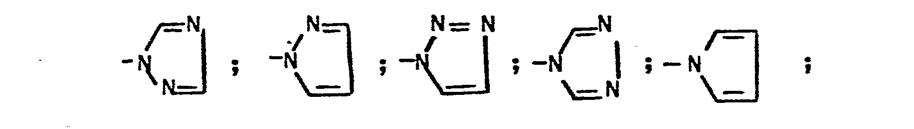 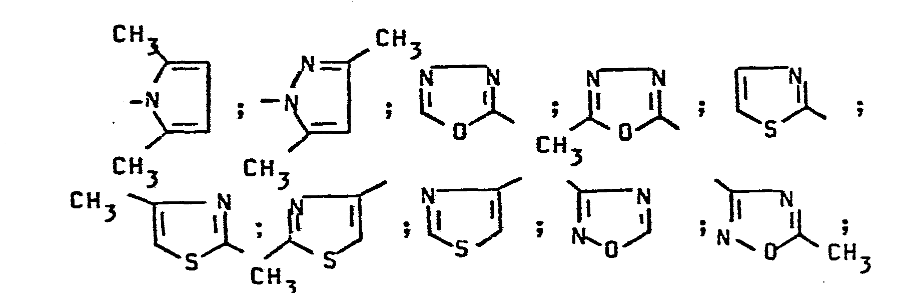 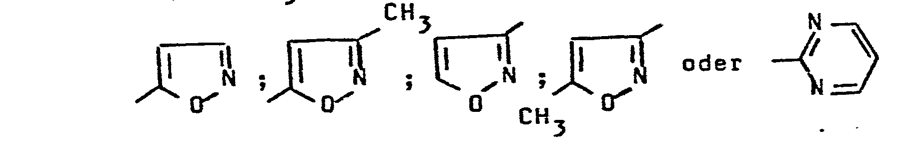 steht.

5. Verfahren zur Herstellung von 1-Aryl-4-heterocyclyl- pyrazolen der Formel (I), 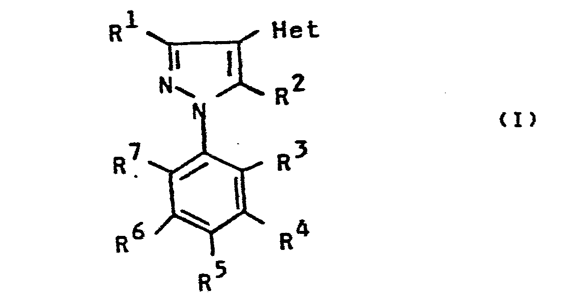 in welcher
R¹ für Wasserstoff oder Alkyl steht,
R² für Wasserstoff oder Halogen steht,
R³,R⁴,R⁵,R⁶ und R⁷ unabhängig voneinander jeweils für Wasserstoff, Cyano, Nitro, Halogen, Alkyl, Alkoxy, Alkoxycarbonyl, Halogenalkyl, Halogenalkoxy oder für einen Rest -S(O)ₙ-R⁸ stehen, mit der Maßgabe, daß mindestens einer der Reste _{R}³, _{R}⁴, _{R}⁵, _{R}⁶ oder R⁷ verschieden von Wasserstoff ist, wobei
R⁸ für Alkyl, Halogenalkyl, Amino, Alkylamino oder Dialkylamino steht und
n für eine Zahl 0, 1 oder 2 steht
und
Het für einen gegebenenfalls durch Halogen, Nitro, Alkyl, Alkoxy, Alkylthio oder Halogenalkyl substituierten aromatischen Ein-Ring-Heterocylus steht,
dadurch gekennzeichnet, daß man 5-Amino-1-aryl-4- heterocyclyl-pyrazole der Formel (II), in welcher 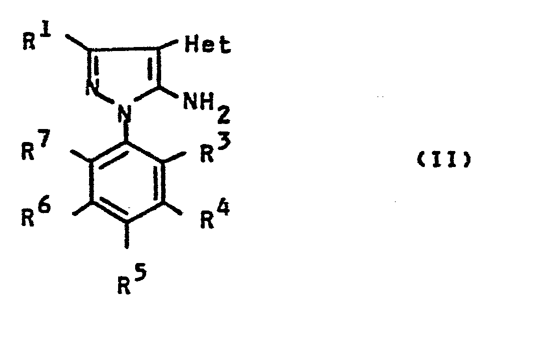
R¹,R³,R⁴,R⁵,R⁶,R⁷ und Het die oben angegebene Bedeutung haben,
mit einem anorganischen oder organischen Nitrit in Gegenwart eines Reaktionshilfsmittels und gegebenenfalls in Gegenwart einer Halogenwasserstoffsäure, sowie gegebenenfalls in Gegenwart eines Reduktionsmittels und gegebenenfalls in Gegenwart eines Verdünnungsmittels umsetzt.

6. Herbizide Mittel, gekennzeichnet durch einen Gehalt an mindestens einem 1-Aryl-4-heterocyclyl-pyrazol der Formel (I) gemäß. den Ansprüchen 1 bis 5.

7. Verfahren zur Bekämpfung von Unkräutern, dadurch gekennzeichnet, daß man ein 1-Xryl-4-heteracyclyl-pyrazol der Formel (I) gemäß den Ansprüchen 1 bis 5 auf Unkräuter undloder ihren Lebensraum einwirken läßt.

8. Verwendung von l-Aryl-4-heterocyclyl-pyrazolen der Formel (I) gemäß den Ansprüchen 1 bis 5 zur Bekämpfung von Unkräutern.

9. Verfahren zur Herstellung von herbiziden Mitteln, dadurch gekennzeichnet, daß man 1-Aryl-4-hetero- cyclylpyrazole der Formel (I) gemäß den Ansprüchen 1 bis 5 mit Streckmitteln und/oder oberflächenaktiven Mitteln vermischt.
